# EUROPÄISCHE PATENTSCHRIFT

(11) **EP 0 421 981 B1**
(45) Veröffentlichungstag und Bekanntmachung des Hinweises auf die Patenterteilung: **09.03.1994**
(21) Anmeldenummer: 91100480.2
(22) Anmeldetag: 11.07.1987
(51) Int. Cl.: A61M 39/00

(54) **Kathetervorrichtung**
Catheter device
Dispositif de cathéter

(30) Priorität: 21.08.1986 DE 3628337
(43) Veröffentlichungstag der Anmeldung: 10.04.1991
(62) Teilanmeldung aus: 87110053.3
(73) Patentinhaber: B. Braun Melsungen AG, 34209 Melsungen (DE)
(72) Erfinder: Stöber, Herbert, W-3513 Staufenberg 1 (DE); Brencher, Norbert, W-3435 Hess. Lichtenau (DE)
(74) Vertreter: Selting, Günther, Dipl.-Ing.

(56) Entgegenhaltungen:
- DE-A- 3 102 142
- DE-U- 8 434 177
- US-A- 4 592 749

## Beschreibung

Die Erfindung betrifft eine Kathetervorrichtung nach dem Oberbegriff des Patentanspruches 1.

Für gewisse Therapien ist eine ständige Zufuhr von Pharmazeutika in den Körper eines Patienten erforderlich. Zu diesem Zweck wird ein Ende des Katheters in ein Blutgefäß oder ein Organ eingeführt und das andere Ende des Katheters wird mit der Anschlußeinheit, z.B. einer Kapsel oder einer Spritze, klemmend verbunden.

Bei der erwähnten bekannten Kathetervorrichtung (DE-A- 31 02 142), sind zur Erzielung eines schnellen, jedoch leicht lösbaren Ankuppelns des Katheters der Anschlußstutzen und das Druckstück durch Schraubgewinde miteinander verbindbar und das elastomere Klemmstück klemmt durch radiale Verformung den Katheter im Innern des Anschlußstutzens fest. Dabei besteht die Gefahr, daß das Material des Klemmstückes sich bei der axialen Zusammenpressung unkontrolliert örtlich radial so verlagert, daß der weiche Katheter bis zum Verschluß seines Lumens zusammengequetscht wird, wenn das Druckstück in einer für eine ausreichende Abdichtung notwendig erscheinenden Weise auf den Anschlußstutzen aufgeschraubt worden ist.

Aus US-A-4 592 749 ist eine Katheteranschlußvorrichtung bekannt, bei der ein radial aufweitbarer Katheter aus Silikongummi auf einen rohrförmigen Dorn größeren Durchmessers aufgesteckt ist, so daß die radiale Spannkraft des aufgeweiteten Katheterendes eine gewisse Haltekraft zur Befestigung des Katheters an einer Anschlußeinheit hervorruft. Zur Verbesserung der Befestigung ist auf das aufgeweitete Katheterende eine Hülse aus hartem Polyethylen aufgeschoben, die ihrerseits umgeben wird von einer flexiblen Manschette, die einen Ringbund der Anschlußeinheit zur Sicherung gegen axiales Abziehen hintergreift. Dieser Koaxialaufbau erfüllt seine Aufgabe nur bei radial aufweitbaren Kathetermaterialien, weil die starre Hülse einen nicht durch Aufweitung bereits auf einem Dorn festsitzenden Katheter nicht festzuspannen vermag. Außerdem ist das Übereinanderschieben der mehreren rohrförmigen Teile umständlich und erlaubt kein schnelles, jedoch leicht lösbares Ankuppeln des Katheters an eine Anschlußeinheit, das beispielsweise günstig ist, wenn sich während einer Operation zeigt, daß der Katheter am patientenfernen Ende gekürzt werden muß.

Der Erfindung liegt die Aufgabe zugrunde, eine Kathetervorrichtung zu schaffen, bei der ein schnelles, zuverlässig dichtes, jedoch leicht lösbares Ankuppeln des Katheters an den Anschlußstutzen ohne Zusammenquetschen des Katheters erreicht wird. Die Lösung dieser Aufgabe erfolgt erfindungsgemäß mit den Merkmalen des Patentanspruchs 1.

Nach der Erfindung ist die Stützkanüle in demjenigen Abschnitt des Katheters vorgesehen, auf dem das Klemmstück, das zum Fixieren des Katheters axial zusammengepreßt wird, sitzt. Die Stützkanüle, die aus einem dünnwandigen starren Rohr, vorzugsweise aus Metall, besteht, stützt den Katheter von innen her ab und verhindert das Einschnüren des Katheterlumens durch das radial nach innen drückende Klemmstück.

Die Stützkanüle ist fest an der Anschlußeinheit angebracht und ragt koaxial in den Anschlußstutzen hinein bzw. durch diesen hindurch. Der Außendurchmesser der Stützkanüle entspricht dem Innendurchmesser des Katheters, so daß der Endabschnitt des Katheters leicht auf die Stützkanüle aufgeschoben werden kann.

Die Verformung des elastomeren Klemmstücks erfolgt vorzugsweise durch zwei als Innenkonus ausgebildete Stirnflächen, von denen die eine an einem Stützflansch der Stützkanüle und die andere am Druckstück angeordnet ist. In den Bereichen der konischen Stirnflächen ergibt sich eine verstärkte radiale Anpressung des Klemmstücks an den Katheter, so daß zwei hintereinander angeordnete Dichtflächen an den beiden Enden des Klemmstücks erzeugt werden.

Im folgenden wird unter Bezugnahme auf die Zeichnungen ein Ausführungsbeispiel der Erfindung näher erläutert.

Es zeigen:
- Fig. 1: einen Längsschnitt durch eine Kapsel mit angeschlossenem Katheterende und
- Fig. 2: eine Draufsicht der Kapsel nach Fig. 1.

Die Kathetervorrichtung weist eine Kapsel (Anschlußeinheit) 10 und einen Katheter 11 in Form eines flexiblen Schlauchs auf, dessen Ende an einen Hohlraum 12 im Innern der Kapsel 10 angeschlossen ist. Der Hohlraum 12 dient zur Aufnahme eines flüssigen Medikaments, das durch den Katheter 11 in ein Gefäß eines Patienten eingeleitet werden kann. Die Kapsel 10 weist eine Bodenwand 13, eine Umfangswand 14 und eine perforierbare Oberwand 15 auf. Diese Wände 13, 14 und 15 umschließen den Hohlraum 12. Die Oberwand 15 besteht aus einer Membran, die mit einer (nicht dargestellten) Hohlnadel durchstochen werden kann und die sich nach dem Herausziehen der Hohlnadel wieder abdichtend schließt.

Das Gehäuse der Kapsel 10 besteht aus zwei Gehäuseteilen 16 und 17. Das innere Gehäuseteil 16 bildet die Bodenwand 13 und den unteren Teil der Umfangswand 14, und das äußere Gehäuseteil 17 umgibt das innere Gehäuseteil 16 umfangsmäßig und überragt dieses Gehäuseteil nach oben. Am oberen Ende weist das Gehäuseteil 17 einen ringförmigen wulstartigen Kragen 18 auf, der von oben her gegen den Rand der Oberwand 15 drückt und diesen Rand gegen die obere Stirnseite des inneren Gehäuseteils 16 gedrückt hält. Die Gehäuseteile 16 und 17 sind miteinander verklebt oder verschweißt. Der wulstförmige Kragen 18 bildet eine trichterförmige Begrenzung der Oberwand 15, wodurch das Auffinden der Oberwand und das Durchstechen mit der Hohlnadel erleichtert wird. Die Kapsel 10 wird unter der Haut des Patienten implantiert, wobei die Oberwand 15 der Haut zugewandt, also nach außen gerichtet ist. Der Rand der Oberwand 15 ist stufenförmig abgesetzt, so daß der obere Teil der Oberwand 15 in den von dem Kragen 18 umschlossenen Bereich hinein nach oben vorsteht.

Das äußere Gehäuseteil 17, das in Draufsicht kreisrund ist, weist an seinem unteren Ende einen radial abstehenden ringförmigen Flansch 19 auf, der mit Löchern 20 zum Fixieren der Kapsel an der Faszie versehen ist. Der Flansch 19 umgibt die Bodenwand 13 des Hohlraums 12 und befindet sich in der gleichen Ebene wie die Bodenwand. Die Gehäuseteile 16 und 17 sind passend so ineinandergesetzt, daß sie spielfrei (ohne Hohlräume) ineinanderpassen und daß die Kapsel 10 eine kontinuierliche ebene Unterseite aufweist.

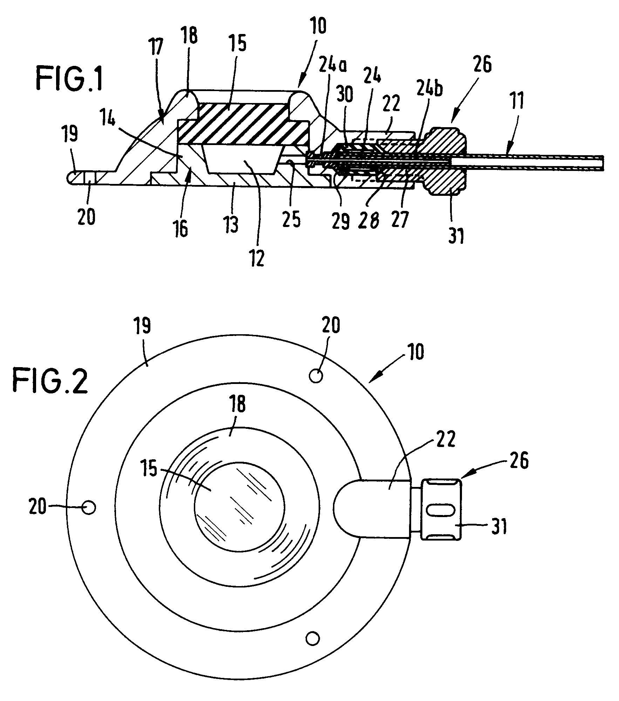

Der Anschlußstutzen 22 ist dem äußeren Gehäuseteil 17 einstückig angeformt. Dieser Anschlußstutzen erhebt sich von dem Flansch 19 nach oben und geht in den Sei tenwandbereich des äußeren Gehäuseteils 17 über. Wie insbesondere aus Fig. 2 zu ersehen ist, steht der Anschlußstutzen 22 nicht über den Flansch 19 hinaus seitlich vor, so daß er die radialen Abmessungen der Kapsel nicht vergrößert.

Der Anschlußstutzen 22 weist einen zylindrischen Klemmraum auf, dessen Umfangswand auf einem Teil der Länge von dem Innengewinde 23 gebildet wird. In den Anschlußstutzen 22 führt der Katheter 11 hinein. In das festzulegende Katheterende ragt die Stützkanüle 24 hinein. Diese Stützkanüle weist einen Befestigungsabschnitt 24a auf, der an der Kapsel 10 fixiert, z.B. von dem Kunststoffmaterial des Gehäuseteils 17 umspritzt, ist, und einen geradlinigen rohrförmigen Stützabschnitt 24b, der in dem Anschlußstutzen 22 koaxial verläuft und unabgestützt nach außen ragt. Die Stützkanüle 24 weist einen von einem Ende zum anderen durchgehenden Längskanal auf, der über eine Bohrung 25 des Gehäuseteils 16 mit dem Hohlraum 12 verbunden ist.

Auf den Katheter 11 ist das Druckstück 26 aufgeschoben, das einen Längskanal aufweist, in dem der Katheter 11 leicht verschiebbar ist. Das Druckstück 26 ist mit einem Gewindeschaft 27 versehen, dessen Außengewinde mit dem Innengewinde 23 zusammengreift. Am rückwärtigen Ende des Gewindeschafts 27 befindet sich der Drehkopf 31, der Greifrippen aufweist, um gedreht zu werden. Der Stützabschnitt 24b der Stützkanüle 24 erstreckt sich im Katheter 11 bis in das Druckstück 26 hinein.

In der vorderen Stirnwand des Gewindeschafts 27 des Druckstücks 26 ist eine trichterförmige Vertiefung 28 vorgesehen, die den Klemmraum begrenzt. Die gegenüber liegende Begrenzung des Klemmraums wird von der trichterförmigen Stirnfläche des Stützflansches 29 der Stützkanüle 24 gebildet. Gegen diese Stirnfläche des Stützflansches 29 stößt das Katheterende.

Der Klemmraum wird von dem elastomeren Klemmstück 30, das in Fig. 1 im ungespannten Zustand dargestellt ist, im wesentlichen ausgefüllt. Dieses Klemmstück besteht aus einem rohrförmigen Ring aus elastomerem Material. Durch die Mittelöffnung des Ringes führt der Katheter 11 hindurch, und die Stirnwände des Ringes sind in Anpassung an die trichterförmigen Stirnflächen der Vertiefung 28 und des Stützflansches 29 kegelstumpfförmig ausgebildet.

Wenn das Druckstück 26 in das Innengewinde 23 eingeschraubt wird, wird das Klemmstück 30 axial (bezogen auf den Katheter 11) zusammengedrückt, wobei es sich gleichzeitig radial aufweitet. Die größte radiale Pressung erfolgt im Bereich der trichterförmigen Stirnflächen der Vertiefung 28 und des Stützflansches 29, so daß das Klemmstück 30 an seinen beiden Enden verstärkt gegen den Umfang des Katheters 11 gedrückt wird, während der Bereich zwischen den Enden weniger stark an den Katheter angedrückt wird oder sogar von diesem abhebt. Auf diese Weise werden an den Enden des Klemmstücks 30 zwei Druck- und Abdichtbereiche geschaffen, durch die der Katheter 11 relativ zur Kapsel 10 fixiert wird. Außerdem erfolgt eine Abdichtung des Katheterdurchgangs durch den Anschlußstutzen 20, so daß keine Flüssigkeit außen am Katheter entlang aus dem Hohlraum 12 ausfließen kann.

Wie aus Fig. 2 erkennbar ist, ragt nur ein Teil des Druckstücks 26 über die Kontur der Kapsel 10 in radialer Richtung hinaus.

Zum Befestigen des Katheters 11 an der Kapsel 10 wird das Druckstück 26 losgeschraubt, so daß das Klemmstück 30 entlastet ist. Der Katheter 11 wird durch das Druckstück 31 hindurch auf den Stützabschnitt 24b der Stützkanüle 24 aufgeschoben, bis das Katheterende gegen die Stirnfläche 29 stößt. Dann wird das Druckstück 26 festgeschraubt, wobei das Klemmstück 30 die in Fig. 1 dargestellte Form annimmt und den Katheter radial gegen den Stützbereich 24b preßt.

## Patentansprüche

1. Kathetervorrichtung mit einem Katheter (11), dessen eines Ende mit einer Anschlußeinheit (10) verbunden ist, die einen Anschlußstutzen (22) und ein in den Anschlußstutzen eingeführtes Druckstück (26) mit einem Axialkanal für den Katheter (11) aufweist, wobei im Innern des AnschluBstutzens (22) ein den Katheter (11) umgebendes elastomeres Klemmstück (30) enthalten ist, das von dem mit dem Anschlußstutzen (22) zusammenschraubbaren Druckstück (26) axial zusammendrückbar ist und dabei eine radiale Klemmung des Katheters (11) verursacht,
**dadurch gekennzeichnet**,
daβ eine starre rohrförmige Stützkanüle (24) vorgesehen ist, deren Auβendurchmesser dem Innendurchmesser des Katheters (11) entspricht und die mindestens den vom Klemmstück (30) umgebenen Abschnitt des Katheters (11) von innen abstützt, daβ
die Stützkanüle (24) mit einem Befestigungsabschnitt (24a) an der Anschluβeinheit (10) befestigt ist und in Richtung auf das freie Ende des Anschlußstutzens (22) nicht abgestützt koaxial zum AnschluBstutzen (22) nach außen absteht und
daß die Stützkanüle (24) einen Stützflansch (29) mit einer dem Klemmstück (30) zugewandten trichterförmigen Stirnfläche zum Abstützen des Klemmstücks (30) aufweist.

## Claims

1. A catheter device comprising a catheter (11) having one end connected to a connection unit (10) which is provided with a connection piece (22) and a pressing piece (26) guided into said connection piece and including an axial channel for the catheter (11), wherein the interior of the connection piece (22) accommodates an elastomeric clamping piece (30) which surrounds the catheter (11) and is adapted to be axially compressed by the pressing piece (26) provided for screw connection with the connection piece (22), and which thus effects a radial clamping of the catheter (11),
**characterized in**
that there is provided a rigid tubular support cannula (24) having an outer diameter corresponding to the inner diameter of the catheter (11) and, from the inside, supporting at least the portion of the catheter (11) surrounded by the clamping piece (30),
that the support cannula (24) has a fastening portion (24a) fastened to the connection unit (10) and in the direction of the free end of the connection piece (22) projects outwardly coaxially to the connection piece (22) and without being supported, and
that the support cannula (24) is provided with a support flange (29) having a funnel-shaped end face facing the clamping piece (30), for supporting the clamping piece (30).

## Revendications

1. Dispositif de catheter comportant un cathéter (11), dont une extrémité est reliée avec une unité de raccordement (10), qui présente une tubulure de raccordement (22) et une pièce de pression (26) introduite dans la tubulure de raccordement, avec un canal axial pour le cathéter (11), dans lequel, à l'intérieur de la tubulure de raccordement (22), est contenue une pièce de serrage élastomère (30), qui entoure le cathéter (11), peut être comprimée axialement par la pièce de pression (26) pouvant être réunie par vissage avec la tubulure de raccordement (22), et cause un serrage radial du cathéter (11), caractérise en ce
qu'il est prévu une canule de support, rigide, tubulaire (24), dont le diamètre extérieur correspond au diamètre intérieur du cathéter (11) et qui soutient, à partir de l'intérieur, au moins la section du cathéter (11), qui est entourée par 12 pièce de serrage (30),
que la canule de support (24) est fixée avec une section de fixation (24a) à l'unité de raccordement (10) et fait saillie extérieurement en direction de l'extrémité libre de la tubulure de raccordement (22) coaxialement à celle-ci, sans être soutenue, en en ce
que la canule de support (24) présente une collerette de support (29) possédant une surface frontale en forme d'entonnoir, qui est tournée vers la pièce de serrage (30) et sert à supporter la pièce de serrage (30).
